# EUROPEAN PATENT SPECIFICATION

(11) **EP 3 838 147 B1**
(45) Date of publication and mention of the grant of the patent: **06.12.2023**
(21) Application number: 21151736.2
(22) Date of filing: 26.06.2013
(51) Int. Cl.: A61B 5/1468

(54) **SENSOR ELEMENT FOR DETECTING AN ANALYTE IN A BODY FLUID**
SENSORELEMENT FÜR DIE ERKENNUNG EINES ANALYTEN IN EINER KÖRPERFLÜSSIGKEIT
ÉLÉMENT DE CAPTEUR POUR DÉTECTER UN ANALYTE DANS UN FLUIDE CORPOREL

(30) Priority: 29.06.2012 EP 12174251
(43) Date of publication of application: 23.06.2021
(62) Divisional of application: 13731776.4
(73) Proprietor: Roche Diabetes Care GmbH, 68305 Mannheim (DE); F. Hoffmann-La Roche AG, 4070 Basel (CH)
(72) Inventor: ACHMANN, Sabine, 68167 Mannheim (DE); KOTZAN, Holger, 68526 Ladenburg (DE); MÜLLER, Ulrich, 44287 Dortmund (DE); STRASSER, Monika, 68167 Mannheim (DE); WIEDER, Herbert, 68623 Lampertheim (DE)
(74) Representative: Freiherr von Campenhausen, Harald

(56) References cited:
- WO-A1-2007/071562
- US-A1- 2009 198 117
- US-A1- 2010 230 285

## Description

### Field of the invention

The invention refers to a sensor element for determining the concentration of an analyte in a body fluid as well as to a method of manufacturing the sensor element. The sensor element is at least partially implantable into a tissue of a body of a human user or an animal. Sensor elements of this type are generally used in medical diagnostics, specifically in the field of home monitoring, such as for electrochemically determining a concentration of one or more analytes such as glucose, triglycerides, lactate, cholesterol or other types of analytes or analyte combinations.

### Related art

The determination of the concentration of one or more analytes such as glucose, in one or more body fluids, such as interstitial fluid and/or blood, is an essential component of therapy and/or prevention in many diseases. Specifically, the determination of blood glucose concentration as well as a corresponding medication is an essential part of daily routine for many diabetics. In order to increase convenience and in order to avoid restricting the daily routine by more than a tolerable degree, portable devices are known in the art, such as for measuring blood glucose concentration, during work, leisure or other activities away from home. Specifically, electrochemical measurement techniques are known using sensors which are fully or partially implantable into a body tissue of the user and which are capable of providing continuous or discontinuous measurements of the analyte concentration. Examples of these types of implantable sensor elements are disclosed in WO 2007/071562 A1, US 2011/0021889 A1, US 2010/0230285 A1, WO 2005/078424 A1 or by international patent application PCT/EP2011/072732.

Test elements for electrochemically detecting the concentration of at least one analyte in a body fluid, such as for determining a blood glucose concentration in blood and/or interstitial fluid, typically comprise at least one working electrode as well as at least one counter electrode. In addition, optionally, the sensor element may comprise at least one reference electrode. However, in alternative embodiments, a reference electrode may be obleft and/or may be combined with the counter electrode. For potential electrode materials, both for the working electrode and the counter electrode, as well as for potential electrochemical measurement setups for determining the analyte concentration by using corresponding amperometric setups, reference may be made to WO 2007/071562 A1. However, other types of measurement setups are possible, in order to derive an analyte concentration from a comparison of electrode potentials.

In typical electrochemical sensor setups, the counter electrode is provided in order to close the electric circuit to the working electrode. For this purpose, typically, redox currents and/or, to a lower extent, capacitive charging currents are used. Typically, the working electrode comprises at least one detector substance adapted to perform an oxidation reaction or a reduction reaction with the analyte. In many cases, the detector substance comprises at least one enzyme such as glucose oxidase (GOD). In case the detection reaction comprises an oxidation reaction at the working electrode, the counter electrode typically provides a reduction reaction in order to close the electric circuit.

Various electrode setups are known in the art. Thus, in US 2009/0198117 A1, an analyte sensor apparatus for implantation within a mammal is disclosed. The analyte sensor apparatus comprises a base layer, a conductive layer disposed on the base layer, an analyte sensing layer and an analyte modulation layer disposed on the analyte sensing layer. The conductive layer includes a working electrode which comprises a plurality of conductive nanotubes. The analyte sensing layer comprises an oxidoreductase disposed on the conductive nanotubes. The oxidoreductase generates hydrogen peroxide in the presence of an analyte to be sensed. The analyte modulating layer modulates the diffusion of the analyte therethrough.

As mentioned above, a plurality of electrode combinations is known. Thus, in three electrode setups, besides the working electrode and the counter electrode, at least one reference electrode is provided, independently from the counter electrode. The potential of the counter electrode, in this case, typically may be adjusted independently from the potential of the reference electrode. As explained in more detail in WO 2007/071562 A1, a potentiostatic controller may be provided, which, on one hand, provides a desired potential difference or voltage between the working electrode and the reference electrode and, on the other hand, is adapted such that a current of the detection reaction taking place at the working electrode is balanced by an appropriate counter process at the counter electrode, thereby closing the electric circuit. For the latter purpose, the counter electrode has to be adjusted to a potential at which the appropriate and required current is generated by an appropriate electrode reaction at the counter electrode.

Thus, the process taking place at the counter electrode may be compared to the process of galvanostatic potentiometry. The counter electrode generally will attain a potential at which the redox process generates the required current. In case this redox process of one redox system is insufficient for providing an appropriate current, the reaction partners will diminish and the counter electrode will proceed to a potential of a subsequent redox reaction, until the sum of all partial currents is sufficient for generating an appropriate counter current for balancing the detection reaction of the working electrode.

The potential of the counter electrode as well as the electrode reactions at the counter electrode will typically depend on a plurality of factors. Thus, the surface of the counter electrode itself will have an influence, such as the surface area, the roughness of the electrode and/or other surface properties. Further, the presence of redox species and the respective overpotential at the counter electrode will influence the above-mentioned properties of the counter electrode, as well as the concentration of the redox species and the redox potential and overvoltage of the process.

Specifically in the case of in-vivo continuous monitoring sensors, these sensors will typically be surrounded by blood and/or interstitial fluid. As mentioned above, most known biosensors are using oxidation detection reactions at the working electrode in which the analyte, such as glucose, is oxidized. As an example, glucose will enzymatically be oxidized, and reduced co-products will be generated, such as H₂O₂. Since most constituents of blood and interstitial fluid are present in a reduced form, the number of reducible species in typical in-vivo measurements is limited. In the order of their respective redox potentials, the following reducible species may be named as examples: oxygen, H₂O₂, H₂O. The amount of oxygen is typically rather limited, specifically in in-vivo measurements, specifically in interstitial fluid. By encapsulation of the sensor element implanted into the body tissue, a delivery of oxygen to the working electrode may further diminish over time. H₂O₂ may be generated by electrode reaction, such as by reduction of O₂ and/or by enzymatic reaction. H₂O typically is widely available at high concentrations. However, using water as a reducible species typically includes a formation of H₂-gas. This gas formation may lead to a de-wetting of the electrode, which, typically, will increase the above-mentioned effects. Further, the formation of gas may lead to a lift-off of a membrane which typically covers the working electrode and may even lead to a full removal of the membrane and/or the electrode.

The counter electrode itself may even be made from a reducible material. Thus, redox materials such as Ag/AgCl systems are known in the art, such as for combined counter electrodes/reference electrodes. In these electrode systems, however, the amount of the redox material typically is limited, thereby limiting the life-cycle of the sensor element. In case the amount of the redox material is insufficient, the depletion of the redox material may lead to a failure of the measurement, such as a failure of the reference potential in a two-electrode-setup, with all potential consequences. Thus, a validity of a calibration may fail or, in a worst case, the working electrode even may be destroyed. In an electrode setup comprising, besides at least one working electrode and at least one counter electrode, at least one reference electrode ("three-electrode-setup"), the counter electrode may have to change to a different redox process in case one or more redox materials are used up. In this case, the counter electrode may even change to an invalid redox process for which the counter electrode is not designed, specifically with regard to size and/or geometry. Still, from a practical point of view, it is typically not possible to increase the amount of the redox material at the counter electrode at will. Thus, an increase of the redox material, such as an increase of Ag/AgCl, in order to increase the lifetime and/or capacity of the counter electrode, may lead to a decreased biocompatibility of the sensor element. It is therefore preferable to make use of a redox species which is available in-vivo and, thus, which is per se biocompatible.

### Problem to be solved

It is therefore an objective of the present invention to provide a sensor element, which is at least partially implantable into a body tissue, which fully or partially avoids the shortcomings of known sensor elements as discussed above. Specifically, the sensor element shall provide improvements regarding the problem of unwanted, increased or inhomogeneous formation of gas at one or more of the electrodes as well as the problem of a potential risk of an unwanted change of electrode potentials or even electrode destruction.

### Summary of the invention

This problem is solved by a sensor element with the features of the independent claims. Preferred embodiments of the sensor element and method for manufacturing the sensor element are disclosed in the specification and in the dependent claims.

According to the present invention there is provided a sensor element for determining the concentration of at least one analyte in a body fluid, wherein the sensor element is at least partially implantable into a body tissue, the sensor element having a substrate and at least two electrodes, the electrodes comprising at least one working electrode and at least one counter electrode, wherein the working electrode comprises at least one conductive pad applied to the substrate, wherein at least one electrically conductive sensor material is applied to the conductive pad, the electrically conductive sensor material comprising at least one detector substance adapted to perform an electrically detectable electrochemical detection reaction with the analyte, wherein the counter electrode comprises at least one counter electrode conductive pad applied to the substrate, wherein the sensor element further comprises at least one electrically insulating material, wherein the electrically insulating material surrounds the counter electrode on all sides, wherein a height of the electrically insulating material at least equals the height of the counter electrode conductive pad wherein the electrically insulating material forms one or more depressions, wherein the counter electrode is fully or partially located within the at least one depression.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which a solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or nonoptional features of the invention.

In a first aspect of the invention, a sensor element for determining the concentration of at least one analyte in a body fluid is disclosed, wherein the sensor element is at least partially implantable into a body tissue. The at least one analyte may comprise one or more analytes which typically are present in a body of a human or animal user, such as one or more metabolites. As an example, the at least one analyte may comprise glucose, triglycerides, lactate, cholesterol and/or any other type of potential analyte which may be found in a body fluid. The body fluid itself may preferably comprise blood and/or interstitial fluid. However, other types of body fluids may be used. In a preferred embodiment, without restricting further embodiments, the invention in the following will mainly be disclosed in the context of a blood glucose sensor.

The sensor element is at least partially implantable into a body tissue. As used herein, the term "at least partially implantable" refers to the fact that at least a part of the sensor element, such as a specific front portion of the sensor element, may be implanted into a body tissue of a human or animal user. The term "implantable" refers to the fact that at least the part dedicated to implantation or insertion into the body tissue is biocompatible, at least over a specific time of use, such as over several days, one week or even several weeks or months. Thus, the sensor element may fully or at least partially be encapsulated by a biocompatible membrane, such as one or more of the membranes known from the above-mentioned prior art documents. Thus, as an example, the membrane as disclosed in WO 2007/071562 A1 may be used and/or one or more of the membranes disclosed by WO 2005/078424 A1. However, other methods for rendering the sensor element fully or at least partially biocompatible and, thus, implantable into a body tissue may be realized in addition or alternatively.

The sensor element has at least one substrate and at least two electrodes. As used herein, the term "substrate" refers to a carrier element which, basically, may have an arbitrary shape, such as a strip-shape. Preferably, the at least one substrate is a flexible substrate. Preferably, the substrate comprises a layer setup having one, two or more layers, preferably a flexible layer setup. The substrate may generally be made of any arbitrary substrate material, such as a plastic material and/or a laminate material and/or a paper material and/or a ceramic material. Other materials may be used alternatively or additionally, such as metals or thin-film setups.

As used herein, the term "electrode" refers to an entity of the sensor element which is adapted to get in contact with the body fluid inside the body tissue, either directly or via at least one semipermeable membrane. Thus, preferably, the electrode is arranged such that the electrode gets in contact with at least one electrolyte contained in the body fluid, such as water. The electrode may be or may comprise one or more electrode fields which fully or partially may be or get in contact with the body fluid. Each electrode field thus may provide at least one interface with the body fluid, e.g. Either directly being in contact with a body tissue containing the body fluid or getting into contact with the body fluid via at least one membrane which may fully or partially be permeable for the body fluid or one or more components thereof. One or more of the electrode fields may be contacted via one or more appropriate contact leads, also referred to as conductive paths. Thus, one contact lead or conductive path may electrically contact precisely one electrode or a plurality of two or more electrodes. The at least one electrode preferably may have exactly one continuous surface area which may be adapted to get in contact with a body fluid inside the body tissue. One or more electrodes of the same type may be provided in the sensor element. Each electrode may be contacted electrically by at least one contact lead. In case more than one electrode of the same type is provided, the electrodes may be contacted by one or more contact leads. Thus, two or more electrodes of the same type might be contacted electrically by one and the same contact lead. Alternatively, separate contact leads may be provided for contacting the electrodes, such as at least one separate contact lead per electrode.

Each electrode may be embodied such that an electrochemical reaction may take place at the electrode, wherein the body fluid or a part thereof, such as an electrolyte and/or the analyte, takes place in this electrochemical reaction. Thus, the electrode may be embodied such that an oxidation reaction or a reduction reaction may take place at the electrode.

The electrodes, or at least one of the electrodes, preferably comprise a multi-layer setup, having at least one metal pad and, optionally, at least one additional layer partially or, preferably, fully covering the metal pad. Thus, the working electrode, as disclosed in further detail below, comprises at least one conductive pad, and the counter electrode as disclosed in further detail below comprises at least one counter electrode conductive pad. Additionally, at least one reference electrode may be provided, which may comprise at least one reference electrode conductive pad. The at least one optional additional layer may comprise at least one electrode material as outlined in further detail below. The at least one metal pad preferably may comprise one or more of the following metals: gold, nickel, copper, platinum. However, other types of metals may be used in addition or alternatively. Further, a multi-layer metal setup may be used, such as for improving an adhesion of the metal pad to the substrate.

The metal pad of each electrode may be connected to one or more electrical wires and/or electrical vias and/or to one or more electrical supply lines or conductor lines. Thereby, the conductive pad or metal pad may be connected to at least one contact pad of the sensor element adapted for connecting the sensor element to at least one measurement device, such as a hand-held measurement device interacting with the sensor element.

The electrodes comprise at least one working electrode and at least one counter electrode. In addition, the sensor element may further comprise at least one reference electrode. However, as outlined in detail e.g. in WO 2007/071562 A1, the reference electrode may also be combined with the counter electrode, the counter electrode taking a double function.

As used herein, the term working electrode refers to an electrode being adapted for performing at least one electrochemical detection reaction for detecting the at least one analyte in a body fluid. As further used herein, the term counter electrode refers to an electrode adapted for performing at least one electrochemical counter reaction adapted for balancing a current flow required by the detection reaction at the working electrode. The term reference electrode refers to an electrode adapted for providing a widely constant electrode potential as a reference potential, such as by providing a redox system having a constant electrode potential.

For measurement setups making use of the working electrode and the counter electrode and, optionally, the reference electrode, reference may be made to Figures 3A and 3B of document WO 2007/071562 A1 as well as the corresponding description of these figures. However, other setups are possible.

The working electrode comprises at least one conductive pad applied to the substrate, preferably at least one metal pad, as outlined above. Further, the working electrode comprises at least one electrically conductive sensor material. The at least one electrically conductive sensor material is applied to the conductive pad and comprises at least one detector substance adapted to perform an electrically detectable electrochemical detection reaction with the analyte.

The counter electrode comprises at least one counter electrode conductive pad applied to the substrate.

For potential embodiments of the electrically conductive sensor material, reference may be made to the above-mentioned prior art documents. Thus, preferably, the at least one electrically conductive sensor material may comprise at least one electrically conductive material such as a carbon or manganese dioxide (MnO₂) paste. Preferably, the electrically conductive sensor material may comprise an electrically conductive matrix, such as a matrix made of manganese dioxide (MnO₂). The at least one electrically conductive sensor material additionally or alternatively may comprise other types of conductive materials and/or non-conductive materials. The electrically conductive sensor material comprises at least one detector substance to perform an electrically detectable electrochemical detection reaction with the analyte. Thus, as outlined above, the at least one detector substance may comprise one or more enzymes, such as glucose oxidase (GOD) and/or glucose dehydrogenase (GDH), preferably an enzyme which, by itself and/or in combination with other components of the detector substance, preferably is adapted to perform an oxidation and/or reduction reaction with the at least one analyte to be detected. Again, reference may be made to the above-mentioned prior art documents, such as to WO 2007/071562 A1 and/or the other documents mentioned in the prior art section, with regard to potential further embodiments of the sensor material which may be realized additionally and/or alternatively. The sensor material may further comprise one or more auxiliary components, such as one or more co-enzymes and/or may comprise one or more mediators which may be adapted for an improved charge transfer from one component of the detection reaction to another component. The above-mentioned MnO₂ may also function as a mediator.

As further used herein, the term "electrically detectable", in conjunction with the electrochemical detection reaction, refers to the fact that, by using an electric and/or electronic setup, the electrochemical detection reaction may be detected. Thus, as an example, the electrochemical detection reaction may be detected by comparing one or more currents while the electrode potentials are constant, such as an electrical current of the working electrode, with the electrical current of one or more further electrodes, such as the electrical current of the counter electrode and/or of a reference electrode. Additionally or alternatively, other electrical measurement methods may be used for detecting the electrochemical detection reaction, such as the measurement setups disclosed in WO 2007/071562 A1, such as by using the measurement setups disclosed in Figures 3A and 3B as well as in the corresponding description of these figures of WO 2007/071562 A1.

The sensor element further comprises at least one electrically insulating material. The electrically insulating material may be applied to the substrate, such as by coating the substrate with one or more layers of the at least one electrically insulating material. As an example, electrically insulating material may comprise one or more electrically insulating resins. Thus, the substrate may fully or partially be coated with one or more layers of the electrically insulating resin. Additionally or alternatively, other types of insulating materials fully or partially covering the substrate may be used. The at least one electrically insulating material may be applied directly or indirectly to the substrate, such as by coating techniques. Again, additionally or alternatively, the electrically insulating material may be part of the substrate itself. Thus, the substrate itself may fully or partially be made of at least one electrically insulating material. As an example, the substrate may fully or partially be made of an insulating plastic material such as an insulating polyester and/or may fully or partially be made of an insulating material such as paper and/or an insulating ceramic material.

Generally, as used herein, the term "electrically conductive" refers to an electric conductivity σ, typically given in S/m or 1/Ωm of at least 1 · 10⁰ S/m, preferably of at least 1 · 10³ S/m and, more preferably, of at least 1 · 10⁵ S/m. As further used herein, the term "electrically insulating" refers to an electric conductivity of no more than 1 · 10⁻¹ S/m, preferably of no more than 1 · 10⁻² S/m and, most preferably, of no more than 1 · 10⁻⁵ S/m.

As outlined above, in case a separate insulating material is used, such as an insulating material forming one or more layers on top of the substrate, the electrically insulating material may be applied directly or indirectly to the substrate, such as by forming one or more layers which directly or indirectly contact the substrate and fully or partially cover the substrate. Thus, the electrically insulating material, preferably forming one or more layers of electrically insulating material, may contact the at least one substrate. Additionally or alternatively, in some regions, at least one additional layer may be interposed in between the electrically insulating material and the substrate, such as at least one electrically conductive feed line or other elements of the sensor element.

The electrically insulating material preferably may comprise one or more plastic materials and/or one or more inorganic electrically insulating materials. Thus, as an example, the at least one insulating material may comprise one or more of the following materials: a polycarbonate; a polyimide; a liquid crystal polymer (LCP); a polyurethane; a polystyrene; a polyethylene terephthalate; fiberglass-reinforced epoxy laminate, preferably a flameretardant fiberglass-reinforced epoxy laminate and more preferably FR4; an electrically insulating photoresist.

The electrically insulating material surrounds, preferably circumferentially surrounds, the counter electrode on all sides. As used herein, the term "surrounding" refers to the fact that the electrically insulating material is present along a borderline of a closed area which fully comprises a surface area counter electrode. Thus, in a top view onto the counter electrode, the counter electrode is surrounded by the electrically insulating material on all sides, such as in a plane or on a curved surface, whereas, preferably, a surface of the counter electrode inside an opening of the insulating material remains free. Thus, as outlined in further detail below, the insulating material may form one or more windows, i.e. openings, wherein the counter electrode is visible through the window. The borderline of the window, however, is fully defined by the insulating material. According to the present invention, the electrically insulating material forms one or more depressions, wherein the counter electrode may fully or partially be located within the at least one depression.

Further, a height of the electrically insulating material at least equals or even exceeds the height of the counter electrode conductive pad. Thus, the electrically insulating material may cover the edges of the counter electrode conductive pad. As used herein, the term "height" specifically may refer to a maximum extension of the electrically insulating material or the electrically conductive sensor material, respectively, in a direction perpendicular to the substrate. Additionally or alternatively, the term height may refer to a height of the counter electrode conductive pad at the edges. Thus, preferably, the counter electrode conductive pad does not protrude to a larger extent from the substrate than the electrically insulating material, at least in the region of the window encompassing the counter electrode.

The sensor element as defined above may further be improved by one or more of the following optional embodiments. Thus, the electrically insulating material, as outlined above, may form at least one layer having at least one opening, wherein the conductive pad may fully or at least partially be located inside the opening. At least the portion of the counter electrode conductive pad located inside the opening preferably may fully be covered by the electrically conductive sensor material. Thus, as outlined above, the insulating material may provide one or more insulating layers having one or more openings, also referred to as "windows", wherein the counter electrode conductive pad at least partially may be visible through these windows.

The insulating material further preferably may cover an edge portion of the counter electrode conductive pad. Thus, in a preferred embodiment, the insulating material may overlap the edges of the counter electrode conductive pad, whereas, inside the window, the counter electrode conductive pad remains free from the insulating material.

Similar embodiments as outlined above for the at least one counter electrode may also be realized for the at least one working electrode. Thus, the electrically insulating material, such as the at least one layer of the electrically insulating material, may have at least one further opening for the working electrode. The conductive pad of the working electrode may fully or partially be located inside the further opening. Thus, as outlined above, one or more windows may be provided in the electrically insulating material, wherein the at least one conductive pad of the working electrode may fully or partially be located inside the window. Additionally or alternatively, the insulating material may comprise one or more depressions, wherein the at least one conductive pad of the at least one working electrode may fully or partially be located inside the at least one depression of the insulating material.

As used herein, the expression "further opening" simply refers to an opening adapted to fully or partially accommodate the working electrode, independent from the opening which may be provided for the at least one counter electrode.

In case the at least one electrically insulating material has a further opening, wherein the conductive pad of the working electrode is fully or partially located within the further opening, the conductive pad of the working electrode, in one embodiment, may be located fully within the further opening. Additionally or alternatively, similar to the preferred embodiment of the counter electrode as outlined above, the at least one conductive pad of the working electrode and the insulating material may be arranged such that the insulating material may cover an edge portion of the conductive pad of the working electrode.

Preferably, at least a portion of the conductive pad located inside the further opening may fully or partially be covered by the electrically conductive sensor material. Further, the at least one insulating material may fully or partially cover an edge portion of the electrically conductive sensor material.

In a further preferred embodiment, the electrically conductive sensor material may be formed by a paste, in a deformable or hardened state. As used herein, the term "paste" refers to an amorphous substance containing one or more particulate components, such as one or more conductive components and/or powders, as well as one or more binder materials, such as one or more organic binder materials. Specifically when using printing techniques, the electrically conductive sensor material may be formed by a printable paste, such as a paste adapted for screen-printing processes.

As further outlined above, at least one of the conductive pad and/or the counter electrode conductive pad preferably comprises at least one metal pad. Thus, the conductive pad of the working electrode and/or the counter electrode conductive pad may fully or partially be made of at least one metal, the at least one metal forming a metal pad. The at least one metal pad may comprise one or more metal layers. In addition or alternatively to at least one metal pad, other types of conductive pads may be used, such as organic conductive pads, such as pads fully or partially formed by conductive polymers. In case one or more metal pads are used, the metal pads preferably comprise at least one gold pad. However, additionally or alternatively, as outlined in further detail above, other types of materials may be used.

The sensor element may comprise precisely one working electrode or may comprise a plurality of working electrodes. Similarly, the sensor element may comprise exactly one counter electrode or may comprise a plurality of counter electrodes. In case one or more additional reference electrodes are provided, precisely one reference electrode may be provided or a plurality of reference electrodes may be provided.

The counter electrode may be formed solely by the counter electrode conductive pad. Thus, a surface of the counter electrode conductive pad may, in an implanted state of the sensor element, be in contact with a body fluid. Thus, a surface of the counter electrode conductive pad may form an electron transfer interface which allows for a charge transfer between the counter electrode conductive pad and the body fluid or vice versa. Thus, electron transfer interface may be an interface allowing for redox reactions.

Additionally or alternatively, the counter electrode conductive pad may fully or partially be covered by at least one electrically conductive counter electrode material. Thus, one or more layers of the electrically conductive counter electrode material may be coated onto the counter electrode conductive pad. In this case, a surface of the electrically conductive counter electrode material facing away from the counter electrode conductive pad may form an electron transfer interface and may be in contact with a body fluid when the sensor element is in an implanted state. In this case, a charge transfer between the electrically conductive counter electrode material and the body fluid or vice versa may take place, and/or redox reactions may take place at the interface between the electrically conductive counter electrode material and the body fluid.

In case the counter electrode further comprises at least one electrically conductive counter electrode material, such as in case the counter electrode conductive pad is covered by one or more layers of the electrically conductive counter electrode material, the height of the electrically insulating material preferably at least equals the height of the electrically conductive counter electrode material. Thus, preferably, the electrically conductive counter electrode material does not protrude from the electrically insulating material.

In case the counter electrode comprises at least one electrically conductive counter electrode material fully or partially covering the counter electrode conductive pad, the at least one electrically conductive counter electrode material may be located fully inside an opening of the insulating material and/or may be fully located inside a depression formed within the electrically insulating material. Additionally or alternatively, the electrically insulating material may cover an edge portion of the electrically conductive counter electrode material.

In case at least one electrically conductive counter electrode material is provided, generally, an arbitrary electrically conductive material or a mixture of electrically conductive materials may be used. In a preferred embodiment, the electrically conductive counter electrode material may comprise at least one of: a paste, preferably a conductive paste, more preferably a carbon paste; an ink, preferably a conductive ink, more preferably a carbon ink.

As outlined above, a surface of the counter electrode being in contact with a body fluid when the sensor element is in an implanted state may form an electron transfer interface or a part of an electron transfer interface. In case the counter electrode conductive pad is fully or partially uncovered by electrically conductive counter electrode material and in case the counter electrode conductive pad is fully or partially in contact with the body fluid when the sensor element is in an implanted state, the uncovered portion of the counter electrode conductive pad being in contact with the body fluid may form the electron transfer interface or a part of the electron transfer interface. In case the counter electrode conductive pad is fully or partially covered by at least one electrically conductive counter electrode material, a surface of the electrically conductive counter electrode material facing away from the counter electrode conductive pad and being in contact with the body fluid when the sensor element is in an implanted state may form the electron transfer interface or a part of the electron transfer interface.

Most preferably, the electron transfer interface does not protrude from the electrically insulating material. Thus, preferably, the height of the electrically insulating material at least equals a height of the electron transfer interface. As used herein, the height of the electron transfer interface may be defined as the maximum distance of the electron transfer interface protruding from the substrate. Thus, as an example, in a direction perpendicular to the substrate, preferably, no point of the electron transfer interface protrudes further from the substrate than the electrically insulating material. Preferably, the electron transfer interface is fully located within an opening of the electrically insulating material and/or within a depression within the electrically insulating material. Again, electrically insulating material may fully or partially cover an edge portion or define an edge of the electron transfer interface.

The sensor element, as outlined in further detail above, may fully or partially be covered by one or more layers improving a biocompatibility of the sensor element or at least of a part of the sensor element, such as of an implantable part of the sensor element. Thus, the sensor element at least partially may be covered by at least one semi-permeable membrane, the semi-permeable membrane preventing the detector substance from migrating into the body fluid. The semi-permeable membrane may further be permeable to the analyte. Further, the semi-permeable membrane may further be permeable to at least one electrolyte contained in the body fluid, such as to water. With regard to potential membrane materials, again, reference may be made to the prior art documents cited above, such as to WO 2007/071562 A1, to WO 2005/078424 A1 or to US 2010/0230285 A1. However, other types of membrane materials may be used, specifically polymer membrane materials, such as polyelectrolyte materials and/or hydrogel-materials.

Further embodiments may refer to the counter electrode. As outlined above, the counter electrode may comprise at least one electrically conductive counter electrode material. The electrically insulating material preferably surrounds the electrically conductive counter electrode material on all sides. Therein, preferably, the height of the electrically insulating material at least equals or even exceeds the height of the electrically conductive counter electrode material. For further details of this geometric setup, reference may be made to the setup of the working electrode disclosed above, wherein the geometric details may be applied, mutatis mutandis, to the counter electrode material and the counter electrode, too.

The electrically conductive counter electrode material may further comprise at least one counter electrode redox material adapted to perform at least one redox reaction and/or may comprise one or more electrically conductive materials such as one or more metals. As used herein, the term redox reaction refers to the fact that one partner of the redox reaction is reduced, whereas another partner of the redox reaction is oxidized. Thus, the term redox material refers to a material comprising at least one reducible component and at least one oxidizable component. As an example, the counter electrode redox material may comprise one or more of the following redox systems: Ag/AgCl; Hg/HgCl₂. The at least one electrically conductive material may comprise one or more of the following electrically conductive materials: Au; Pt; Pd; Carbon. Therein, the at least one electrically conductive material such as one or more of Au, Pt, Pd and Carbon preferably is used in 3-electrode systems, only. The at least one electrically conductive material may provide at least one electrically conductive surface which, preferably, is an electrically polarizable surface, preferably is adapted such that one or more redox reactions with one or more components contained in the body fluid may take place, such as one or more of the following redox reactions: O₂/H₂O; H₂O₂/H₂O; H₂O/H₂. However, other embodiments are possible.

Similarly, as outlined above, the sensor element may further comprise at least one reference electrode. As outlined above, the at least one reference electrode may be combined with the at least one counter electrode (also referred to as a "two-electrode-setup", independently from the number of working electrodes, counter electrodes/reference electrodes). Alternatively, the at least one reference electrode may be formed by at least one separate electrode, independently from the at least one counter electrode (also referred to as a "three-electrode-setup", independently from the number of working electrodes, counter electrodes and reference electrodes).

Again, the reference electrode may comprise at least one reference electrode conductive pad, preferably at least one reference electrode metal pad. For potential details, reference may be made to the above-mentioned optional embodiments of the counter electrode conductive pad. The reference electrode may further comprise at least one electrically conductive reference electrode material. The electrically conductive reference electrode material preferably may comprise at least one reference redox material providing a known redox potential. As used herein, the term "providing a known redox potential" refers to the fact that the reference redox material is capable of performing a redox reaction the potential of which is precisely defined. Additionally or alternatively, one or more other types of electrically conductive materials may be comprised, such as one or more metals and/or carbon.

In case the sensor element comprises at least one reference electrode, the layer of the electrically insulating material may have at least one of the further opening for the reference electrode, wherein the reference electrode may be located at least partially inside the further opening. The above-mentioned geometric details of the insulating material may also refer to the at least one reference electrode. Thus, the electrically insulating material may surround the electrically conductive reference electrode material on all sides. Therein, preferably, the height of the electrically insulating material at least equals or even may exceed the height of the electrically conductive reference electrode material.

The reference redox material preferably may comprise one or more redox systems having a precisely defined redox potential. Similarly to the counter electrode, the at least one redox material preferably may comprise one or more of the following redox systems: Ag/AgCl; Hg/HgCl₂; Hg/Hg₂SO₄; Hg/HgO. Additionally or alternatively, other redox systems may be employed.

The sensor element, as outlined above, may generally have an arbitrary shape. Preferably, the sensor element may have an elongated shape having a length and a width. Thus, the sensor element may have a longitudinal axis or axis of longitudinal extension, along which the sensor element may extend. The sensor element may be a rigid sensor element or may be flexible or deformable. Preferably, the length of the sensor element exceeds the width by at least a factor of 10, preferably by at least a factor of 20. Thus, preferably, the sensor element may be a needle-type sensor element and/or may comprise a sensor element strip.

Along the longitudinal axis of extension, the sensor element may extend into the body tissue. The sensor element preferably may have a length of 0.5 to 100 mm, such as a length of 5 mm to 20 mm and, more preferably, a length of 13 mm. However, other lengths are possible, such as lengths below 13 mm. In use, the sensor element may extend into the body by preferably more than 3 mm, such as by 7 mm to 10 mm or even more.

The width of the sensor element, such as a width perpendicular to a longitudinal axis of extension, preferably may be around 0.1 mm to around 10 mm, preferably around 0.2 mm to around 1 mm, such as 0.4 mm or 0.7 mm. However, generally, a width of less than 0.5 mm, such as a width of less than 0.4 mm, is possible.

The working electrode and the counter electrode generally may be applied to the same surface of the substrate or to different surfaces of the substrate. Thus, in one embodiment of the present invention, the working electrode and the counter electrode may be applied to opposing surfaces of the substrate. Again, at least one working electrode may be located on a first side of the substrate and at least one counter electrode may be located on an opposing side of the substrate. Thus, preferably, the substrate may have an elongated flat shape, wherein on one side of the elongated flat shape, the at least one working electrode is applied to the substrate and wherein, on an opposing side, the at least one counter electrode is applied to the substrate. Preferably, in case the working electrode and the counter electrode are applied to opposing surfaces of the substrate, a projection of the working electrode into a plane of the counter electrode is overlapped by the counter electrode. Thus, when projecting the working electrode and the counter electrode into a common plane, such as a plane of extension of the sensor element and/or a plane of extension of the substrate, the counter electrode preferably fully overlaps the projection of the working electrode. Thus, the projection of the working electrode may fully be located inside the projection of the counter electrode.

Further preferred aspects of the sensor element refer to a symmetry of the location of the working electrode and the counter electrode. Thus, preferably, the sensor element may be embodied such that the working electrode and the counter electrode are applied to opposing surfaces of the substrate, wherein projections of the working electrode and the counter electrode into a common plane, such as into a plane of the substrate, are arranged essentially symmetrical with respect to at least one axis of symmetry. Thus, one, two or more axes of symmetry may be present, which may function as mirror axes. Thus, the projections of the working electrode and of the counter electrode into the common plane may be symmetrical with regard to one and the same axis of symmetry and/or with regard to two axes of symmetry. Specifically, a mirror symmetry may be present. The at least one axis of symmetry may comprise at least one longitudinal extension of the sensor element, i.e. a longitudinal axis of extension of the sensor element and/or an axis which is parallel to this axis of longitudinal extension of the sensor element. As used herein, the term "essentially symmetrical" refers to a perfect symmetry, wherein, within the tolerances of manufacturing, slight deviations from a perfect symmetry may be possible, preferably deviations by no more than 1 mm, preferably by no more than 0.5 mm and more preferably by no more than 0.02 mm. Additionally or alternatively to the longitudinal extension of the sensor element, a symmetry may exist with regard to at least one axis perpendicular to the lateral extension of the sensor element. Further details will be outlined below with regard to specific embodiments of the sensor element.

Thus, as an example, the sensor element may comprise at least two counter electrodes which are arranged on opposing sides of the working electrode or, in case a plurality of working electrodes is provided, of at least one of the working electrodes. Thus, as will be outlined in further detail below, the substrate may have an elongated shape with an axis of longitudinal extension. Along the axis of longitudinal extension, firstly, a counter electrode may be arranged, followed by a working electrode, followed again by another counter electrode. Further electrodes may be present. The counter electrodes may be substantially equally spaced apart from the working electrode. As used herein, substantially equally spaced apart generally refers to the fact that the distances between the counter electrodes and the working electrode are equal within the tolerances of manufacturing. Thus, within the tolerances of manufacturing, the smallest distance between the first counter electrode and the working electrode and the smallest distance between the second counter electrode and the working electrode may be identical. As used herein, the smallest distance between the counter electrode and the working electrode may be a distance between an edge of the counter electrode closest to the working electrode and an edge of the working electrode closest to the counter electrode. With regard to potential tolerances of manufacturing, reference may be made to the preceding paragraph. Thus, generally, the working electrode may symmetrically be surrounded by at least two counter electrodes.

As outlined above, the substrate preferably may have an elongated shape having an axis of longitudinal extension. The sensor element may comprise at least two counter electrodes, wherein, in a direction of longitudinal extension of the sensor element, one of the counter electrodes is located on each side of the working electrode, wherein the counter electrodes are equally spaced apart from the working electrode. Thus, two or more counter electrodes may be located symmetrically on both sides of the working electrode.

Additionally or alternatively, with the substrate having an elongated shape with an axis of longitudinal extension, the sensor element may also comprise at least two working electrodes, wherein, in a direction of the longitudinal extension of the sensor element, one of the working electrodes is located on each side of the counter electrode, wherein the working electrodes are equally spaced apart from the counter electrode. Thus, two or more working electrodes may be located symmetrically on both sides of the counter electrode.

The preferred symmetrical placement of at least two counter electrodes with regard to a working electrode and/or the preferred symmetrical placement of at least two working electrodes with regard to a counter electrode may be realized such that the electrodes are placed within placement tolerances. Thus, placement tolerances of less than 100 µm in each direction are preferred, more preferably placement tolerances of no more than 50 µm in each direction and, most preferably, placement tolerances of the electrodes of no more than 20 µm in each direction.

As outlined above, the substrate preferably may have an elongated shape having an axis of longitudinal extension. The sensor element may have a width in a dimension perpendicular to this axis of longitudinal extension. Preferably, in this direction of the width of the sensor element, the working electrode and the counter electrode each are equally spaced apart from at least two lateral edges of the substrate. Thus, the working electrode may equally be spaced apart from a first lateral edge of the substrate and a second lateral edge of the substrate. Similarly, the counter electrode may equally be spaced apart from the first lateral edge of the substrate and the second lateral edge of the substrate. Thus, in both directions perpendicular to the axis of longitudinal extension, the working electrode may equally be spaced apart from the lateral edges of the substrate.

Similarly, in these two directions perpendicular to the axis of longitudinal extension, the counter electrode may equally be spaced apart from the lateral edges of the substrate. Further details will be outlined below with regard to specific embodiments of the invention.

In a further aspect of the present disclosure, a method for manufacturing the sensor element of the invention, in one or more of the embodiments disclosed above or disclosed in further detail below, is disclosed. In this method of manufacturing, the electrically conductive sensor material is applied to the conductive pad by at least one coating technique. Thus, the method may comprise one or more coating steps, using the at least one coating techniques, wherein during the at least one coating step the electrically conductive sensor material is applied to the conductive pad. Thereby, the conductive pad may fully or partially be covered by the electrically conductive sensor material.

Similarly, in case the at least one counter electrode further comprises at least one electrically conductive counter electrode material which at least partially covers the counter electrode conductive pad, the method may comprise at least one step of applying the electrically conductive counter electrode material to the counter electrode conductive pad by using at least one coating technique.

The at least one coating technique preferably may comprise one or more of the following techniques: a printing technique; a dispensing technique. The printing technique may comprise an arbitrary printing technique, such as screen printing, in-jet printing, stencil printing, offset printing, tampon printing or any other printing technique or any arbitrary combination thereof. Additionally or alternatively, other coating techniques may be employed.

The electrically conductive sensor material may be applied in an amorphous form, such as a paste or liquid, preferably followed by at least one drying step. Other types of coating techniques may be employed additionally or alternatively.

Thus, the method may comprise further steps, such as at least one step of providing the at least one substrate, at least one further step of providing the at least one conductive pad and/or the at least one counter electrode conductive path on the substrate and, finally, the above-mentioned coating step wherein the electrically conductive sensor material is applied to the conductive pad and/or wherein the electrically conductive counter electrode material is applied to the counter electrode conductive pad by at least one coating technique, such as by at least one printing technique and/or at least one dispensing technique. In case a printing technique is used, the at least one printing technique may comprise one or more different types of printing techniques. Preferably, the printing technique comprises at least one screen printing technique. The electrically conductive sensor material and/or the electrically conductive counter electrode material, as outlined above, preferably may be applied as a paste. The electrically conductive sensor material and/or the electrically conductive counter electrode material may further be coated into one or more openings of the electrically insulating material, such as by at least one of a printing technique and a dispensing technique, such as by screen printing. Additionally or alternatively, the insulating material may be applied after coating, such as after printing and/or dispensing, of the electrically conductive sensor material and/or the electrically conductive counter electrode material, respectively.

The sensor element according to the present invention provide a large number of advantages over known sensor elements and manufacturing techniques. Thus, the counter electrode may even fully or partially be made of a non-reducible material. Since, in this case, in vivo only a typical number of reducible reaction partners exists, such as oxygen, H₂O₂ or water, the electrode reaction at the counter electrode will typically imply a gas formation, such as the formation of H₂ gas. By using the present invention, as will be outlined in further detail below, a formation of gas may be realized in a significantly more homogeneous way, such as by homogeneously distributing the gas formation over the counter electrode and, thereby, allowing for an improved removal of this gas formation, such as by diffusion processes. Even a membrane may be used, which fully or partially covers the counter electrode. Thus, one or more membranes may be used for the purpose of immobilizing the conductive sensor material and/or the detector substance, such as for immobilizing an enzyme. Further, the purpose of the membrane may reside in a limitation of a diffusion of the analyte. Further, the overall biocompatibility of the sensor element may be increased by the membrane. By improving the electrode geometry, as outlined above, the membrane may even cover all of the electrodes, such as working electrode, counter electrode and, optionally, the at least one reference electrode, which significantly improves and simplifies the manufacturing techniques. Still, by using the present invention, the gas formation may take place in a more homogeneous way and/or even at a distance from the working electrode and the reference electrode, thereby generally avoiding or at least decreasing the above-mentioned problems of the gas formation, such as problems of de-wetting, interaction with the electrode process, electrical problems or even a lifting-off of the membrane or the electrodes. Generally, gas formation may take place in a more homogeneous way, since, by covering the edges of the electrodes with the electrically insulating material, the overall electric fields at the electrodes may be designed in a significantly more homogeneous way as compared to conventional sensor elements. Thereby, at a given current, at each point of the counter electrode the same amount of gas may be generated and removed. The electrically insulating material may form part of the substrate itself, which may fully or partially be made of the electrically insulating material. Additionally or alternatively, the at least one electrically insulating material may be applied to the substrate, such as by coating the substrate with one or more layers of the electrically insulating material. Thus, as outlined above or as outlined in further detail below, the electrically insulating material may comprise at least one electrically insulating resin.

The present invention is specifically advantageous in conjunction with elongated sensor elements, such as so-called needle-type sensors, and/or in conjunction with other in-vivo-sensors having a complex sensor geometry. Thus, in these complex sensor geometries, the working electrode and the counter electrode typically are provided in a non-ideal geometry, strongly deviating from a plane-parallel geometry of the electrode surfaces generating a homogeneous electric field. Thus, specifically, in needle-type sensors, electrode configurations are used having significantly complex structures of the electric fields, having contorted electric fields and/or electric fields of a locally high density. Thus, the density of the electric flux lines significantly may be high in the region of corners, edges or other parts of the electrodes, leading to high variations in voltage drop at the counter electrode. By using the above-mentioned electrode geometry which fully or partially covers these edges by the insulating material surrounding the electrically conductive sensor material of at least the working electrode and, more preferably, also of the counter electrode, a more homogeneous distribution of the electric field may be provided. Consequently, by avoiding local areas having a high density of the lines of electric flux, the electrode reactions may be designed in a significantly more homogeneous way, thereby leading to a more homogeneous formation of gas.

Thereby, in conjunction with an appropriate measurement technique, such as by using one or more potentiostatic measurements for a two-electrode-setup or a three-electrode-setup, the above-mentioned problems may be avoided. As an example, the measurement setup disclosed in WO 2007/071562 A1 may be used. By rendering the electrode reactions, specifically at the counter electrode, more homogeneous, the potentiostatic setup may control the potential of the counter electrode such that a homogeneous current distributed over the electrode surface will flow, the current having a significantly increased homogeneity as compared to conventional sensor elements with uncovered corners and/or edges of the electrodes. Again, a more homogeneous formation of gas may take place. Consequently, by using the present invention, a gas formation under the membrane and/or a de-wetting of the counter electrode, causing a significantly increased current density on a decreasing surface, may be avoided. Similarly, disturbations of the current path in between the working electrode and the counter electrode may be avoided as well as disturbations of transport processes at the working electrode and the counter electrode.

Further advantages of the present invention refer to sensor elements having a plurality of working electrodes and/or a plurality of counter electrodes. Thus, changing geometries, such as sensor elements having alternating arrangements of working electrodes and counter electrodes, may be realized. In conventional devices, these changing geometries may lead to an unwanted situation in which the current flows over only one out of a plurality of potential current paths, thereby leading to a localized gas formation and/or other localized problems. By using the above-mentioned invention, thereby providing a more homogeneous distribution of the electric fields and/or current paths, these problems may be avoided.

Consequently, a plurality of working electrodes may be provided on the same sensor perimeter, thereby e.g. allowing for a tissue averaging of the measurement process, preferably without changing an angle of implantation of the sensor element. Specifically, by covering the edges of the working electrode and, optionally, the counter electrode by using the electrically insulating material, a direct extension of electric flux lines from the edge of the working electrode to the edge of the counter electrode or vice versa may be avoided, even if the working electrode and the counter electrode are located adjacent to each other on the same surface of the sensor element. Consequently, only curved electric flux lines are possible, thereby providing a more homogeneous electric field, avoiding edge effects and leading to a more homogeneous distribution of the current density.

### Short description of the figures

Further potential details and optional features of the invention may be derived from the following description of preferred embodiments. In these preferred embodiments, the features of the embodiments might be realized in an isolated way or in any arbitrary combination. The invention is not restricted to the preferred embodiments. The embodiments are schematically depicted in the figures. Identical reference numbers in the figures refer to identical or functionally identical elements.

In the figures:
- Figure 1: shows a cross-sectional view of a first embodiment of a sensor element according to the present disclosure;
- Figures 2A and 2B: show in a top view (Figure 2A) and in a cross-sectional view (Figure 2B) a gas formation and a homogeneous distribution of electric flux lines in the sensor element according to the present disclosure;

- Figures 3A and 3B,: in a similar view to Figures 2A and 2B, show the problem of inhomogeneous gas formation in conventional sensor elements without edge covering of the electrodes;
- Figure 4: shows an embodiment of the present invention having a plurality of working electrodes and a plurality of counter electrodes, with a preferred distribution of gas formation;

- Figure 5: shows the problem of inhomogeneous gas formation in embodiments without edge covering of the electrodes;
- Figure 6: shows a multi-layer setup of a sensor element with electrodes on opposing sides;
- Figure 7: shows a different embodiment of a sensor element having sensor electrodes on opposing sides;
- Figures 8A to 8E: show different views of a further embodiment of a sensor element having electrodes on opposing sides;
- Figure 9: shows an embodiment of a sensor element according to the present invention having an insulating material overlapping conductive materials of the working electrode and the counter electrode; and
- Figures 10 and 11: show embodiments of a sensor element having sensor electrodes on opposing sides.

### Preferred embodiments

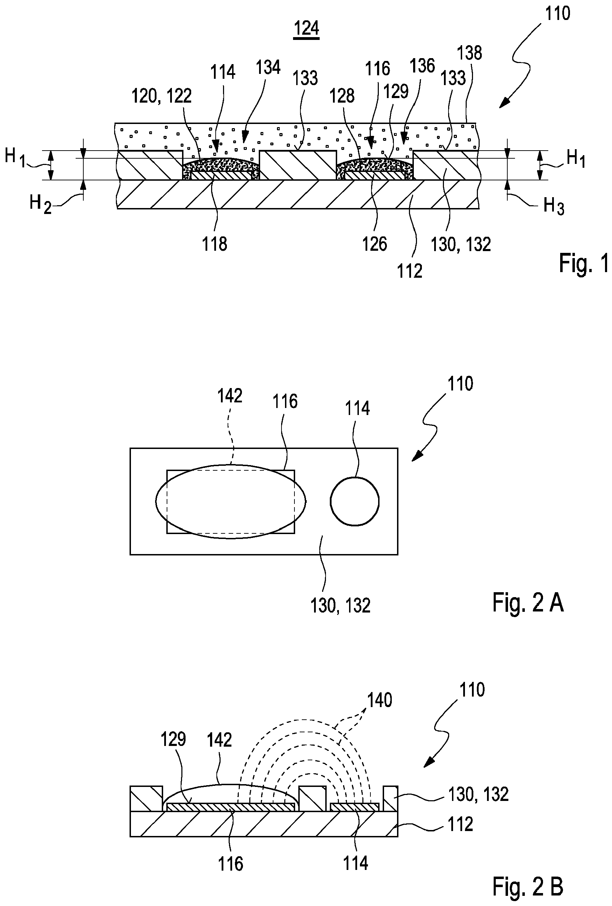

In Figure 1, a cross-sectional view of a first embodiment of a sensor element 110 according to the present invention is shown. The cross-section may be shown in a cross-sectional plane along a longitudinal axis of extension of the sensor element 110.

The sensor element 110 may comprise at least one substrate 112. The sensor element 110 further comprises at least one working electrode 114 and at least one counter electrode 116. The working electrode 114 comprises a working electrode conductive pad 118, such as a metal pad, and at least one electrically conductive sensor material 120 which fully or partially covers the working electrode conductive pad 118. The electrically conductive sensor material 120 comprises at least one detector substance 122, as outlined in further detail above. The detector substance 122 is adapted to perform an electrically detectable electrochemical detection reaction with an analyte to be detected by the sensor element 110 in a body fluid 124 surrounding the sensor element 110, such as a body fluid 124 comprised in a body tissue of a human or an animal user.

The counter electrode 116 comprises at least one counter electrode conductive pad 126. Further, the counter electrode 116 may comprise one or more electrically conductive counter electrode materials 128, such as Ag/AgCl or Hg/HgCl₂. However, embodiments without electrically conductive counter electrode material 128 are feasible. In any case, a surface of the counter electrode 116 facing away from the substrate 112 may be in contact with a body fluid or a part thereof, when the sensor element 110 is in an implanted state. This uppermost surface of the counter electrode, in an implanted state, may form an electron transfer interface, which, in Figure 1, is denoted by reference number 129. In the embodiment depicted in Figure 1, the upper surface of the electrically conductive counter electrode material 128 or a part thereof, which may get in contact with the body fluid, forms the electron transfer interface 129. In embodiments in which no electrically conductive counter electrode material 128 is deposited on top of the counter electrode conductive pad 126, the electron transfer interface 129 may be formed by the top surface of the counter electrode conductive pad 126. Other embodiments are feasible, such as embodiments in which the counter electrode conductive pad 126 is only partially covered by the electrically conductive counter electrode material 128. In this case, the electron transfer interface 129 may partially be formed by a top surface of the electrically conductive counter electrode material 128 and may partially be formed by a surface area of the counter electrode conductive pad 126 which is not covered by the electrically conductive counter electrode material 128.

The sensor element 110 further comprises at least one electrically insulating material 130, which forms one or more electrically insulating layers 132. The electrically insulating layer 132 comprises a plurality of openings 134, 136, also referred to as a "window", which may define an accessible area of the working electrode 114 and the counter electrode 116, respectively. As schematically depicted in Figure 1, the height of the electrically conductive sensor material 120 and the height of the electrically conductive counter electrode material 128 preferably do not exceed the height of the electrically insulating layer 132. In Figure 1, the height of the electrically insulating material 130 is denoted by Hi, whereas the height of the electrically conductive sensor material 120 is denoted by H₂. As can be seen, H₁ preferably at least equals H₂ or, preferably, exceeds H₂. Similarly, the height H₁ of the electrically insulating material 130 at least equals a height H₃ of the electron transfer interface 129. Thus, the electron transfer interface 129 does not protrude from the electrically insulating material 130, and the electron transfer interface 129 is below a top surface 133 of the electrically insulating material 130 surrounding the counter electrode 116, at least in the region of the respective counter electrode 116. Similarly, preferably, the electrically conductive sensor material 120 does not protrude from the electrically insulating material 130 surrounding the working electrode 114, and a top surface of the electrically conductive sensor material 120 is below a top surface 133 of the electrically insulating material 130 surrounding the working electrode 114, at least in the region of the respective working electrode 114.

The sensor element 110 may further comprise at least one membrane 138 which at least may cover the working electrode 114 and which, as in the embodiment depicted in Figure 1, also may fully or partially cover the counter electrode 116. The membrane 138 is a semipermeable membrane which allows for a diffusion of the analyte from the body fluid 124 to the detector substance 122 and, preferably, for a diffusion of an electrolyte from the body fluid 124 to the working electrode 114 and/or the counter electrode 116, whereas the detector substance 122 and/or the electrically conductive sensor material 120 are kept back in the working electrode 114. Thus, in embodiments with or without membrane, the body fluid 124 or a part thereof may reach the counter electrode 116. In the context of the present invention, no distinction is made between embodiments in which the body fluid 124 may fully reach the counter electrode 116 and embodiments in which part of the body fluid 124 is retained by the membrane 138, whereas other parts of the body fluid 124 reach the counter electrode 116.

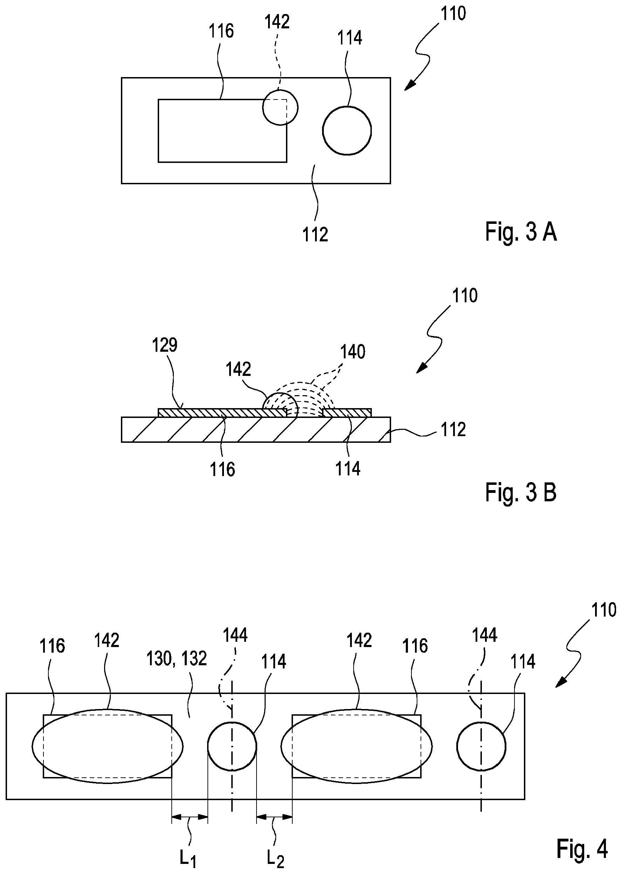

In Figures 2A to 3B, the effect of the present invention is depicted. Thus, Figures 2A and 3A show a top view of a sensor element 110 according to the present invention (Figure 2A) with the electrically insulating material 130 and of a sensor element 110 without the electrically insulating material 130 (Figure 3A). Similarly, Figure 2B shows a side view of Figure 2A, and Figure 3B shows a side view of Figure 3A.

In Figures 2B and 3B, electrical flux lines 140 are schematically depicted for both cases. Thus, in the sensor element 110 according to the present invention as depicted in Figure 2B, at the edges of the electrodes 114, 116, the electric flux lines 140 are blocked by the electrically insulating material 130. Thereby, edge effects with a high density of the electric flux lines 140 are prevented, as opposed to the situation in Figure 3B without the electrically insulating material 130. In this case, which does not form an embodiment of the present invention, a high density of electric flux lines 140 may be recognized in between the edges of the electrodes 114, 116.

Consequently, a formation of gas, denoted by reference number 142, may be different in both cases. Thus, in the setup according to the present invention as depicted in Figures 2A and 2B, a homogeneous formation of gas 142 may take place, which may evenly be distributed across the counter electrode 116. Contrarily, in the setup of Figures 3A and 3B, which do not form an embodiment of the present invention, a gas formation may take place in a rather punctual manner, providing a huge gas bubble rather than an evenly distributed gas film over the counter electrode 116. This formation of gas 142 as depicted in Figure 3A, generating one or more localized gas bubbles, may lead to a destruction of one or more of the electrodes 114, 116 and/or to a lift-off of the membrane 138 (not depicted in these figures).

Thus, a comparison of Figures 2A, 2B and Figures 3A and 3B clearly shows the effect of the present invention rendering the density of the electric flux lines 140 and/or the density of the electric current across the electrodes 114, 116 more homogeneous, thereby homogeneously distributing the formation of gas 142 and preventing a destruction of the sensor element 110 or other problems of inhomogeneous gas formation, as outlined in further detail above.

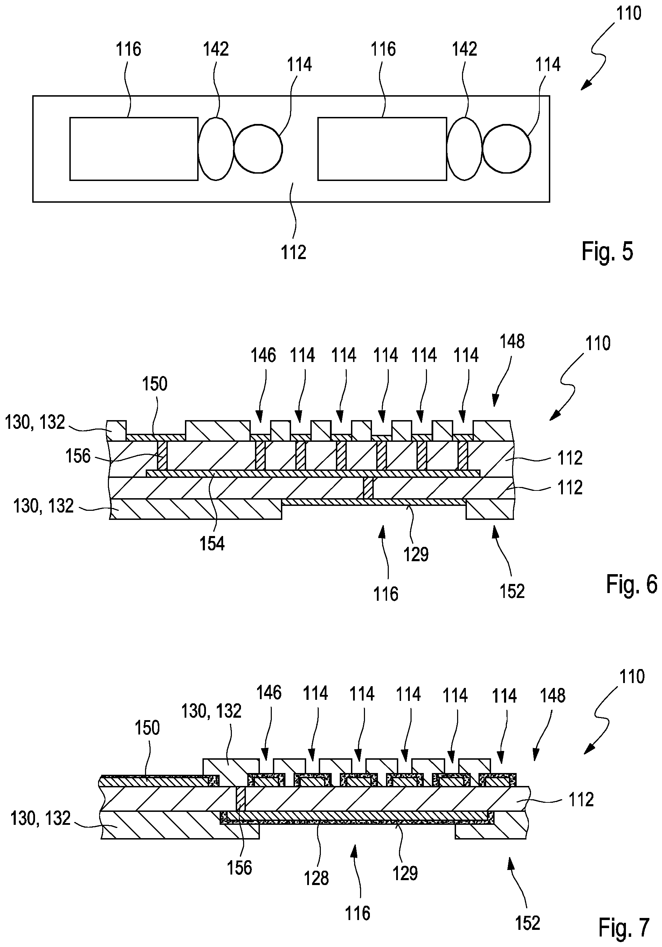

In Figures 4 and 5, a setup of a sensor element 110 having a plurality of alternating working electrodes 114 and counter electrodes 116 is depicted, once in a setup according to the present invention (Figure 4) and in a setup without the electrically insulating material 130 (Figure 5) and, thus, not corresponding to the present invention. For details of a cross-sectional view, reference may be made to Figures 2A to 3B above.

The sensor element 110 according to the present invention as depicted in Figure 4, besides the electrically insulating layer 132, further shows a symmetry of two counter electrodes 116 with regard to an axis of symmetry 144. Thus, at least the left one of the working electrodes 114 preferably is equally spaced apart from two counter electrodes 116, one on the left hand side of axis of symmetry 144 and one on the right hand side of axis of symmetry 144. In addition to the effect of the electrically insulating material 130 as outlined above, this symmetry further enhances a uniformity of the current density and/or the electric flux lines 140 (not depicted in these figures). Thus, in the symmetric setup of Figure 4, a uniform distribution of gas formation 142 takes place since electric flux lines 140 are evenly distributed to the right and to the left. Contrarily, in the asymmetric setup according to Figure 5, a localized gas formation 142 in between a working electrode 114 and the nearest counter electrode 116 takes place. Thus, by locating at least two counter electrodes symmetrically with regard to a corresponding working electrode and/or by locating at least two working electrodes symmetrically with regard to one counter electrode, thereby introducing a specific symmetry, in this or other embodiments of the present invention a more uniform gas formation may be evoked.

Further, the setup of the sensor element 110 as shown in Figure 4 shows an embodiment of two counter electrodes 116 being arranged on opposing sides of one working electrode 114. Further, preferably, the counter electrodes 116 are substantially equally spaced apart from the working electrode 114. Thus, the counter electrodes 116 may be equally spaced apart from the working electrode 114 or from one of the working electrodes 114, within the tolerances of manufacturing. Thus, generally, deviations by no more than 1 mm, preferably by no more than 0.5 mm and more preferably by no more than 0.02 mm may be tolerated. When referring to the distances, reference is made to the smallest distances of the respective electrodes 114, 116. In Figure 4, the distance between the left counter electrode 116 and the working electrode 114 is denoted by Li, whereas the distance between the right counter electrode 116 and the working electrode 114 is denoted by L₂. Within the tolerances of manufacturing, preferably, L₁ equals L₂.

The distance between the working electrode 114 and the counter electrode 116is defined by the minimum distance of the facing edges of the respective working electrode 114 and the counter electrode 116.

Furthermore for the case that more than one working electrode 114 is arranged on the substrate, preferably on the same side of the substrate, each working electrode 114 is neighbored by a respective pair of counter electrodes 116 and the distances L₁, L₂ between each working electrode 114 and the respective neighbored pair of counter electrodes 116 are equal within the tolerance of fabrication for each working electrode. In particular all of the working electrodes 114 - counter electrodes distances on the substrate are equal within the tolerance of fabrication.

In Figure 6, a cross-sectional side view of a further embodiment of a sensor element 110 according to the present invention is depicted. In this embodiment, a reference electrode 146 and a plurality of working electrodes 114 are located on a first surface 148 of a substrate. Further, at least one contact pad 150 may be located on this first surface 148. On a second surface 152 opposing the first surface 148, at least one counter electrode 116 may be provided. Both the working electrodes 114, the reference electrode 146 and the counter electrode 116 are circumferentially surrounded by an electrically insulating material 130. In Figure 6, the electrically conductive sensor material 120 is not shown. Further, the optional electrically conductive counter electrode material 128 is not shown. Thus, the electrodes 114 and 116 are only shown in a schematic fashion.

The sensor element 110 according to Figure 6 may further comprise a multi-layer setup comprising an inner layer of conducting paths 154 and a plurality of electrical vias 156 connecting the conducting paths 154 to the appropriate electrodes 114, 116, 146 or to the contact pad 150.

In addition to the coverage of the edges of the electrodes 114, 116 by the electrically insulating material 130, as disclosed in the context of the embodiments disclosed above, the location of the electrodes 114, 116 on opposing surfaces 148, 152 of the substrate 112 further prevents a direct edge-to-edge interconnection of the edges of the electrodes 114, 116 by electric flux lines 140 and, thus, prevents a localized high current density at these edges, in conjunction with a prevention of a localized increase of gas formation. Consequently, by using electrodes on opposing surfaces of the substrate 112, the above-mentioned effect of a more homogeneous formation of gas on one or more of the electrodes 114, 116 may further be supported. Further, by placing electrodes 114 and 116 on opposing surfaces 148 and 152, the distance between the working electrodes 114 and the counter electrodes 116 may be increased. Thereby, the above-mentioned effect may further be emphasized, and gas formation by increased electrical fields may be avoided by spatially separating electrodes 114 and 116 as far as possible.

In Figure 7, a layer setup of a sensor element 110 according to the present invention is shown in a similar view as compared to Figure 6, including slight modifications of the setup. Thus, instead of using a multi-layer substrate 112, a single layer substrate 112 may be used, including one or more vias 156. Thus, the layer setup may significantly be simplified in terms of complexity. Again, as in Figure 6 disclosed above, the counter electrode 116 may optionally comprise an electrically conductive counter electrode material 128.

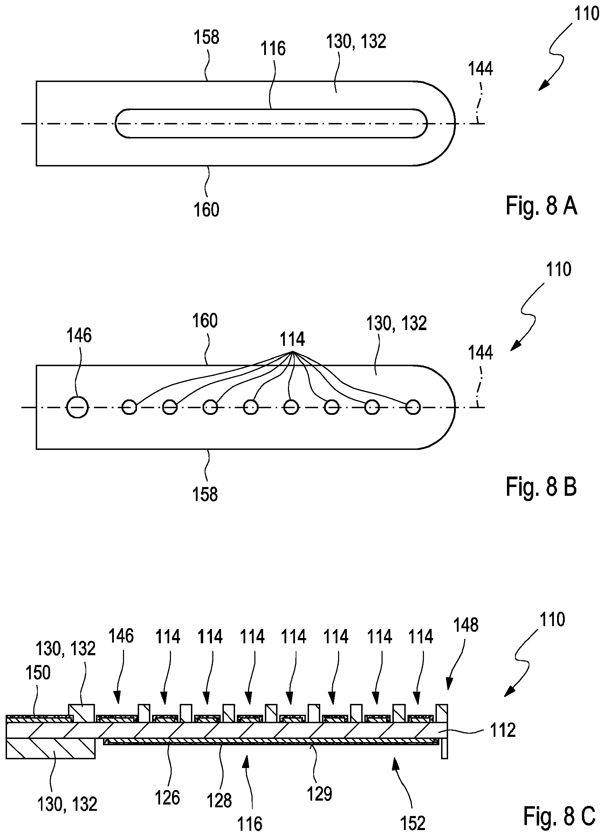

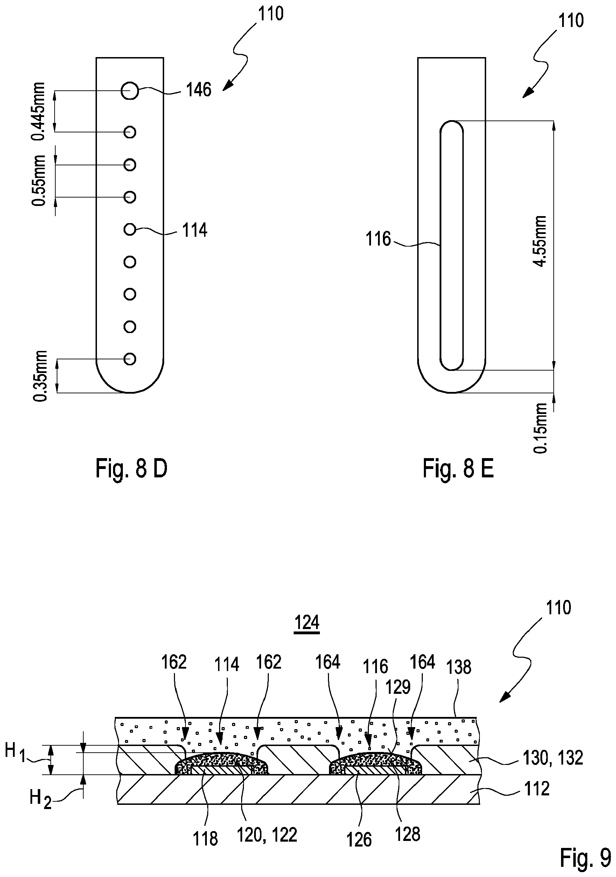

In Figures 8A to 8E, different views of a potential setup of a sensor element 110 according to the present invention are shown, such as the sensor element 110 according to the embodiment depicted in Figure 7. Therein, Figure 8C shows a cross-sectional view, whereas Figures 8A and 8E show a top view of the second surface 152 including the counter electrode. Figures 8B and 8D show a top view of the first surface 148, including the reference electrode 146 and the working electrodes 114. Figures 8D and 8E, additionally, show some potential dimensions of the setup.

Firstly, as can be seen by comparing Figures 8A and 8B, all electrodes in this embodiment are arranged symmetrically with regard to an axis of symmetry 144, which, in this embodiment, preferably runs parallel to an axis of longitudinal extension of the sensor element 110. Thus, counter electrode 116 preferably is equally spaced apart from both lateral edges 158, 160 of the sensor element 110, and, similarly, electrodes 114 are equally spaced apart from these lateral edges 158, 160. As explained above with regard to the symmetry in Figure 4, this measure of introducing a symmetry in the location of the electrodes 114, 116 may further help to render the electric fields, the density of the electric flux lines 140 and the current densities more homogeneous over the electrodes 114, 116, thereby preventing a strongly localized formation of gas.

Further, specifically in case a gas formation takes place at the counter electrode 116, the counter electrode 116, in a projection of the working electrodes 114 and the counter electrode 116 into a common plane, may overlap the working electrodes 116 on all sides. Thus, when projecting the working electrodes 114 into a plane of the counter electrode 116, the working electrodes 114 preferably are fully located inside the counter electrode 116. More preferably, the counter electrode 116, in this projection, in each direction of the plane, overlaps the edges of the working electrodes 114 by preferably at least 100 µm, preferably by at least 200 µm.

The above-mentioned placements and the symmetries shall be understood within specific tolerances of placement, which, typically, are determined by manufacturing and layout techniques. Thus, preferably, the at least one working electrode 114 and the at least one counter electrode 116 are placed within placement tolerances of no more than 100 µm, more preferably within placement tolerances of no more than 50 µm and, most preferably, within placement tolerances of no more than 20 µm.

In Figure 9, a further embodiment of a sensor element 110 according to the present invention is depicted. At first glance, the sensor element 110 corresponds to the embodiment as depicted in Figure 1, so that reference may be made to the description of Figure 1 above. However, deviating from the embodiment of Figure 1, the electrically insulating material 130 overlaps the electrically conductive sensor material 120 in an edge region 162, also referred to as an overlap region, thereby further covering the edges of the working electrode 114 and preventing the above-mentioned unwanted edge effects. Similarly, the electrically insulating material 130 may overlap part of the counter electrode 116, in an edge region 164, specifically the electrically conductive counter electrode material 128.

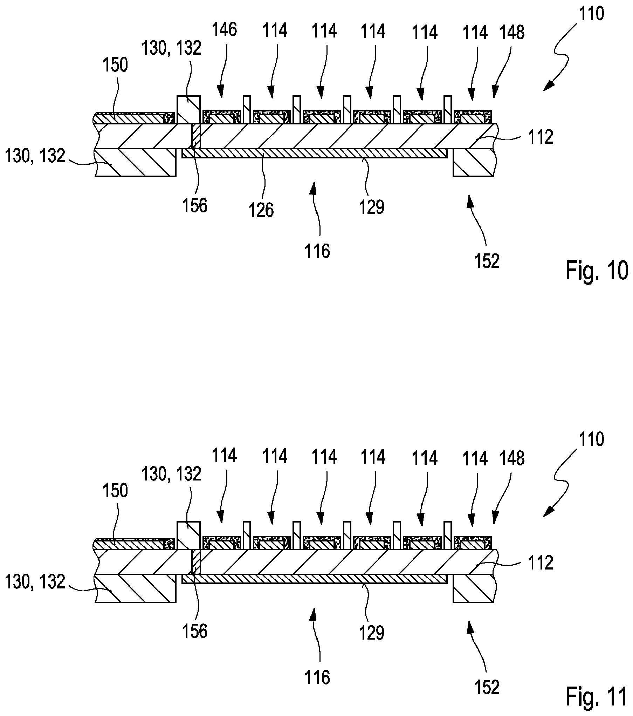

In Figure 10, a modification of the amendment shown in Figure 8C is depicted. Again, sensor element 110 according to Figure 10 comprises a layer setup having the working electrodes 114 and the reference electrode 146 on a first surface 148 of the substrate 112, and the at least one counter electrode 116 on the opposing, second surface 152. As can be seen in Figure 10, the counter electrode conductive pad 126 may be uncovered by electrically conductive counter electrode material 128. Thus, the free surface of the counter electrode conductive pad 126 may form the electron transfer interface 129 which, in an implanted state, gets in contact with the body fluid 124 and/or a part thereof. Further, in this embodiment, the edges of the electrodes 114, 116 and 146 may remain free and uncovered by the electrically insulating material 130. Further, similar to the embodiments shown in Figures 6 and 7, one or more vias 156 may be provided penetrating the substrate 112, thereby e.g. allowing to electrically contact counter electrode 116 on the first surface 148.

In Figure 11, a modification of the embodiment shown in Figure 10 is depicted. In this embodiment, the at least one reference electrode 146 is replaced by at least one further working electrode 114, on the first surface 148. Further, as in Figure 7, the at least one counter electrode 116 on the second surface 152 may be embodied without an electrically conductive counter electrode material 128, as depicted in Figure 11, or may be embodied with an electrically conductive counter electrode material 128, as depicted in Figure 7.

These embodiments clearly demonstrate that various geometric setups are feasible. The design details of the embodiments may be combined in any feasible way, as the skilled person immediately will recognize.

In all embodiments, such as the embodiment depicted in Figure 1 and the embodiment depicted in Figure 11, the electrically conductive sensor material 120 may fully or partially be applied to the substrate 112 by using at least one coating technique, such as by using at least one printing technique (such as screen printing and/or other types of printing techniques) and/or by using at least one dispensing technique. Thus, the electrically conductive sensor material 120 may be formed by at least one paste which may be applied to the substrate 112 by a screen printing technique and/or a stencil printing technique and/or by a dispensing technique. Other types of coating techniques, such as other types of printing techniques, may be applied additionally or alternatively.

### List of reference numbers

- 110: sensor element
- 112: Substrate
- 114: working electrode
- 116: counter electrode
- 118: working electrode conductive pad
- 120: electrically conductive sensor material
- 122: detector substance
- 124: body fluid
- 126: counter electrode conductive pad
- 128: electrically conductive counter electrode material
- 129: Electron transfer interface
- 130: electrically insulating material
- 132: electrically insulating layer
- 133: Top surface
- 134: opening
- 136: opening
- 138: membrane
- 140: electric flux lines
- 142: formation of gas
- 144: axis of symmetry
- 146: reference electrode
- 148: first surface
- 150: contact pad
- 152: second surface
- 154: conducting paths
- 156: Via
- 158: lateral edge
- 160: lateral edge
- 162: edge region
- 164: edge region

## Claims

1. A sensor element (110) for determining the concentration of at least one analyte in a body fluid (124), wherein the sensor element (110) is at least partially implantable into a body tissue, the sensor element (110) having a substrate (112) and at least two electrodes (114, 116), the electrodes (114, 116) comprising at least one working electrode (114) and at least one counter electrode (116), wherein the working electrode (114) comprises at least one conductive pad (118) applied to the substrate (112), wherein at least one electrically conductive sensor material (120) is applied to the conductive pad (118), the electrically conductive sensor material (120) comprising at least one detector substance (122) adapted to perform an electrically detectable electrochemical detection reaction with the analyte, wherein the counter electrode (116) comprises at least one counter electrode conductive pad (126) applied to the substrate (112), wherein the sensor element (110) further comprises at least one electrically insulating material (130), wherein the electrically insulating material (130) surrounds the counter electrode (116) on all sides, wherein a height of the electrically insulating material (130) at least equals the height of the counter electrode conductive pad (126) **characterized in that** the electrically insulating material forms one or more depressions, wherein the counter electrode is fully or partially located within the at least one depression.

2. The sensor element (110) according to the preceding claim, wherein the electrically insulating material (130) forms at least one layer (132) having at least one opening (134, 136), wherein the counter electrode conductive pad (126) is located at least partially inside the opening (134, 136).

3. The sensor element (110) according to any of the preceding claims, wherein the sensor element (110) comprises a plurality of working electrodes (114).

4. The sensor element (110) according to any of the preceding claims, wherein the counter electrode (116) further comprises at least one electrically conductive counter electrode material (128), wherein the at least one electrically conductive counter electrode material (128) at least partially covers the counter electrode conductive pad (126).

5. The sensor element (110) according to the preceding claim, wherein the height of the electrically insulating material (130) at least equals the height of the electrically conductive counter electrode material (128).

6. The sensor element (110) according to any of the preceding claims, wherein the counter electrode (116) comprises at least one electron transfer interface, wherein the electron transfer interface (129), in an implanted state of the sensor element (110), is in contact with at least one body fluid, wherein the height of the electrically insulating material (130) at least equals a height of the electron transfer interface (129).

7. The sensor element (110) according to any of the preceding claims, wherein the electrically insulating material (130) surrounds the electrically conductive sensor material (120) on all sides, wherein a height of the electrically insulating material (130) at least equals the height of the electrically conductive sensor material (120).

8. The sensor element (110) according to any of the preceding claims, wherein the counter electrode (116) further comprises at least one electrically conductive counter electrode material (128), the electrically conductive counter electrode material (128) comprising at least one counter electrode redox material adapted to perform at least one redox reaction, wherein the electrically insulating material (130) surrounds the electrically conductive counter electrode material (128) on all sides, wherein the height of the electrically insulating material (130) at least equals the height of the electrically conductive counter electrode material (128).

9. The sensor element (110) according to any of the preceding claims, wherein the sensor element (110) further comprises at least one reference electrode (146), the reference electrode (146) comprising at least one reference electrode conductive pad and at least one electrically conductive reference electrode material, the electrically conductive reference electrode material comprising at least one reference redox material providing a known redox potential.

10. The sensor element (110) according to the preceding claim, wherein the electrically insulating material (130) surrounds the electrically conductive reference electrode material on all sides, wherein the height of the electrically insulating material (130) at least equals the height of the electrically conductive reference electrode material.

11. The sensor element (110) according to any of the preceding claims, wherein the working electrode (114) and the counter electrode (116) are applied to opposing surfaces (148, 152) of the substrate (112).

12. The sensor element (110) according to the preceding claim, wherein a projection of the working electrode (114) into a plane of the counter electrode (116) is overlapped by the counter electrode (116).

13. The sensor element (110) according to any of the preceding claims, wherein projections of the working electrode (114) and the counter electrode (116) into a common plane are arranged essentially symmetrically with respect to at least one axis (144) of symmetry.

14. The sensor element (110) according to any of the preceding claims, wherein the substrate (112) has an elongated shape having an axis of longitudinal extension, wherein, in a direction perpendicular to the axis of longitudinal extension, at least one of the working electrode (114) and the counter electrode (116) are equally spaced apart from at least two lateral edges (158, 160) of the substrate (112).

15. The sensor element (110) according to any of the preceding claims, wherein the sensor element (110) comprises at least two counter electrodes (116) arranged on opposing sides of the working electrode (114), wherein the counter electrodes (116) are substantially equally spaced apart from the working electrode (114).

## Patentansprüche

1. Sensorelement (110) für die Bestimmung der Konzentration mindestens eines Analyten in einer Körperflüssigkeit (124), wobei das Sensorelement (110) mindestens teilweise in ein Körpergewebe implantiert werden kann, wobei das Sensorelement (110) ein Substrat (112) und mindestens zwei Elektroden (114, 116) aufweist, wobei die Elektroden (114, 116) mindestens eine Arbeitselektrode (114) und mindestens eine Gegenelektrode (116) umfassen, wobei die Arbeitselektrode (114) mindestens ein auf das Substrat (112) aufgebrachtes leitfähiges Kissen (118) umfasst, wobei mindestens ein elektrisch leitfähiges Sensormaterial (120) auf das leitfähige Kissen (118) aufgebracht ist, wobei das elektrisch leitfähige Sensormaterial (120) mindestens eine Nachweissubstanz (122) umfasst, die zum Durchführen einer elektrisch nachweisbaren elektrochemischen Nachweisreaktion mit dem Analyten ausgelegt ist, wobei die Gegenelektrode (116) mindestens ein leitfähiges Gegenelektrodenkissen (126), das auf das Substrat (112) aufgebracht ist, umfasst, wobei das Sensorelement (110) ferner mindestens ein elektrisch isolierendes Material (130) umfasst, wobei das elektrisch isolierende Material (130) die Gegenelektrode (116) auf allen Seiten umgibt, wobei eine Höhe des elektrisch isolierenden Materials (130) mindestens gleich der Höhe des leitfähigen Gegenelektrodenkissens (126) ist,
**dadurch gekennzeichnet, dass**
das elektrisch isolierende Material eine oder mehrere Vertiefungen bildet, wobei sich die Gegenelektrode vollständig oder teilweise in der mindestens einen Vertiefung befindet.

2. Sensorelement (110) nach dem vorstehenden Anspruch, wobei das elektrisch isolierende Material (130) mindestens eine Schicht (132) mit mindestens einer Öffnung (134, 136) bildet, wobei sich das leitfähige Gegenelektrodenkissen (126) mindestens teilweise in der Öffnung (134, 136) befindet.

3. Sensorelement (110) nach einem der vorstehenden Ansprüche, wobei das Sensorelement (110) eine Vielzahl von Arbeitselektroden (114) umfasst.

4. Sensorelement (110) nach einem der vorstehenden Ansprüche, wobei die Gegenelektrode (116) ferner mindestens ein elektrisch leitfähiges Gegenelektrodenmaterial (128) umfasst, wobei das mindestens eine elektrisch leitfähige Gegenelektrodenmaterial (128) das leitfähige Gegenelektrodenkissen (126) mindestens teilweise bedeckt.

5. Sensorelement (110) nach dem vorstehenden Anspruch, wobei die Höhe des elektrisch isolierenden Materials (130) mindestens gleich der Höhe des elektrisch leitfähigen Gegenelektrodenmaterials (128) ist.

6. Sensorelement (110) nach einem der vorstehenden Ansprüche, wobei die Gegenelektrode (116) mindestens eine Elektronenübertragungsgrenzfläche umfasst, wobei die Elektronenübertragungsgrenzfläche (129) in einem implantierten Zustand des Sensorelements (110) mit mindestens einer Körperflüssigkeit in Kontakt ist, wobei die Höhe des elektrisch isolierenden Materials (130) mindestens gleich einer Höhe der Elektronenübertragungsgrenzfläche (129) gleicht.

7. Sensorelement (110) nach einem der vorstehenden Ansprüche, wobei das elektrisch isolierende Material (130) das elektrisch leitfähige Sensormaterial (120) auf allen Seiten umgibt, wobei eine Höhe des elektrisch isolierenden Materials (130) mindestens gleich der Höhe des elektrisch leitfähigen Sensormaterials (120) ist.

8. Sensorelement (110) nach einem der vorstehenden Ansprüche, wobei die Gegenelektrode (116) ferner mindestens ein elektrisch leitfähiges Gegenelektrodenmaterial (128) umfasst, wobei das elektrisch leitfähige Gegenelektrodenmaterial (128) mindestens ein Gegenelektrodenredoxmaterial umfasst, das zum Durchführen mindestens einer Redoxreaktion ausgelegt ist, wobei das elektrisch isolierende Material (130) das elektrisch leitfähige Gegenelektrodenmaterial (128) auf allen Seiten umgibt, wobei die Höhe des elektrisch isolierenden Materials (130) mindestens gleich der Höhe des elektrisch leitfähigen Gegenelektrodenmaterials (128) ist.

9. Sensorelement (110) nach einem der vorstehenden Ansprüche, wobei das Sensorelement (110) ferner mindestens eine Referenzelektrode (146) umfasst, wobei die Referenzelektrode (146) mindestens ein leitfähiges Referenzelektrodenkissen und mindestens ein elektrisch leitfähiges Referenzelektrodenmaterial umfasst, wobei das elektrisch leitfähige Referenzelektrodenmaterial mindestens ein Referenzredoxmaterial umfasst, das ein bekanntes Redoxpotential bereitstellt.

10. Sensorelement (110) nach dem vorstehenden Anspruch, wobei das elektrisch isolierende Material (130) das elektrisch leitfähige Referenzelektrodenmaterial auf allen Seiten umgibt, wobei die Höhe des elektrisch isolierenden Materials (130) mindestens gleich der Höhe des elektrisch leitfähigen Referenzelektrodenmaterials ist.

11. Sensorelement (110) nach einem der vorstehenden Ansprüche, wobei die Arbeitselektrode (114) und die Gegenelektrode (116) auf gegenüberliegenden Oberflächen (148, 152) des Substrats (112) aufgebracht sind.

12. Sensorelement (110) nach dem vorstehenden Anspruch, wobei ein Vorsprung der Arbeitselektrode (114) in eine Ebene der Gegenelektrode (116) von der Gegenelektrode (116) überlappt wird.

13. Sensorelement (110) nach einem der vorstehenden Ansprüche, wobei Vorsprünge der Arbeitselektrode (114) und der Gegenelektrode (116) in eine gemeinsame Ebene im Wesentlichen symmetrisch in Bezug auf mindestens eine Symmetrieachse (144) angeordnet sind.

14. Sensorelement (110) nach einem der vorstehenden Ansprüche, wobei das Substrat (112) eine längliche Form mit einer längs verlaufenden Achse aufweist, wobei in einer Richtung senkrecht zu der längs verlaufenden Achse mindestens eine von der Arbeitselektrode (114) und der Gegenelektrode (116) gleich von mindestens zwei Seitenkanten (158, 160) des Substrats (112) beabstandet ist.

15. Sensorelement (110) nach einem der vorstehenden Ansprüche, wobei das Sensorelement (110) mindestens zwei Gegenelektroden (116) umfasst, die auf gegenüberliegenden Seiten der Arbeitselektrode (114) angeordnet sind, wobei die Gegenelektroden (116) im Wesentlichen gleich von der Arbeitselektrode (114) beabstandet sind.

## Revendications

1. Élément de capteur (110) pour la détermination de la concentration d'au moins un analyte dans un fluide corporel (124), dans lequel l'élément de capteur (110) est au moins partiellement implantable dans un tissu corporel, l'élément de capteur (110) ayant un substrat (112) et au moins deux électrodes (114, 116), les électrodes (114, 116) comprenant au moins une électrode de travail (114) et au moins une contre-électrode (116), dans lequel l'électrode de travail (114) comprend au moins une plaque conductrice (118) appliquée sur le substrat (112), dans lequel au moins un matériau de capteur électriquement conducteur (120) est appliqué sur la plaque conductrice (118), le matériau de capteur électriquement conducteur (120) comprenant au moins une substance détectrice (122) adaptée pour réaliser une réaction de détection électrochimique électriquement détectable avec l'analyte, dans lequel la contre-électrode (116) comprend au moins une plaque conductrice de contre-électrode (126) appliquée sur le substrat (112), dans lequel l'élément de capteur (110) comprend en outre au moins un matériau électriquement isolant (130), dans lequel le matériau électriquement isolant (130) entoure la contre-électrode (116) de tous les côtés, dans lequel une hauteur du matériau électriquement isolant (130) est au moins égale à la hauteur de la plaque conductrice de contre-électrode (126)
**caractérisé en ce que**
le matériau électriquement isolant forme une ou plusieurs dépressions, dans lequel la contre-électrode est entièrement ou partiellement située à l'intérieur de l'au moins une dépression.

2. Élément de capteur (110) selon la revendication précédente, dans lequel le matériau électriquement isolant (130) forme au moins une couche (132) ayant au moins une ouverture (134, 136), dans lequel la plaque conductrice de contre-électrode (126) est située au moins partiellement à l'intérieur de l'ouverture (134, 136).

3. Élément de capteur (110) selon l'une quelconque des revendications précédentes, dans lequel l'élément de capteur (110) comprend une pluralité d'électrodes de travail (114).

4. Élément de capteur (110) selon l'une quelconque des revendications précédentes, dans lequel la contre-électrode (116) comprend en outre au moins un matériau de contre-électrode électriquement conducteur (128), dans lequel l'au moins un matériau de contre-électrode électriquement conducteur (128) recouvre au moins partiellement la plaque conductrice de contre-électrode (126).

5. Élément de capteur (110) selon la revendication précédente, dans lequel la hauteur du matériau électriquement isolant (130) est au moins égale à la hauteur du matériau de contre-électrode électriquement conducteur (128).

6. Élément de capteur (110) selon l'une quelconque des revendications précédentes, dans lequel la contre-électrode (116) comprend au moins une interface de transfert d'électrons, dans lequel l'interface de transfert d'électrons (129), dans un état implanté de l'élément de capteur (110), est en contact avec au moins un fluide corporel, dans lequel la hauteur du matériau électriquement isolant (130) est au moins égale à une hauteur de l'interface de transfert d'électrons (129).

7. Élément de capteur (110) selon l'une quelconque des revendications précédentes, dans lequel le matériau électriquement isolant (130) entoure le matériau de capteur électriquement conducteur (120) de tous les côtés, dans lequel une hauteur du matériau électriquement isolant (130) est au moins égale à la hauteur du matériau de capteur électriquement conducteur (120).

8. Élément de capteur (110) selon l'une quelconque des revendications précédentes, dans lequel la contre-électrode (116) comprend en outre au moins un matériau de contre-électrode électriquement conducteur (128), le matériau de contre-électrode électriquement conducteur (128) comprenant au moins un matériau redox de contre-électrode adapté pour réaliser au moins une réaction redox, dans lequel le matériau électriquement isolant (130) entoure le matériau de contre-électrode électriquement conducteur (128) de tous les côtés, dans lequel la hauteur du matériau électriquement isolant (130) est au moins égale à la hauteur du matériau de contre-électrode électriquement conducteur (128).

9. Élément de capteur (110) selon l'une quelconque des revendications précédentes, dans lequel l'élément de capteur (110) comprend en outre au moins une électrode de référence (146), l'électrode de référence (146) comprenant au moins une plaque conductrice d'électrode de référence et au moins un matériau d'électrode de référence électriquement conducteur, le matériau d'électrode de référence électriquement conducteur comprenant au moins un matériau redox de référence fournissant un potentiel redox connu.

10. Élément de capteur (110) selon la revendication précédente, dans lequel le matériau électriquement isolant (130) entoure le matériau d'électrode de référence électriquement conducteur de tous les côtés, dans lequel la hauteur du matériau électriquement isolant (130) est au moins égale à la hauteur du matériau d'électrode de référence électriquement conducteur.

11. Élément de capteur (110) selon l'une quelconque des revendications précédentes, dans lequel l'électrode de travail (114) et la contre-électrode (116) sont appliquées sur des surfaces (148, 152) opposées du substrat (112).

12. Élément de capteur (110) selon la revendication précédente, dans lequel une projection de l'électrode de travail (114) dans un plan de la contre-électrode (116) est recouverte par la contre-électrode (116).

13. Élément de capteur (110) selon l'une quelconque des revendications précédentes, dans lequel les projections de l'électrode de travail (114) et de la contre-électrode (116) dans un plan commun sont agencées essentiellement symétriquement par rapport à au moins un axe (144) de symétrie.

14. Élément de capteur (110) selon l'une quelconque des revendications précédentes, dans lequel le substrat (112) a une forme allongée ayant un axe d'extension longitudinale, dans lequel, dans une direction perpendiculaire à l'axe d'extension longitudinale, au moins l'une de l'électrode de travail (114) et de la contre-électrode (116) est également espacée d'au moins deux bords latéraux (158, 160) du substrat (112).

15. Élément de capteur (110) selon l'une quelconque des revendications précédentes, dans lequel l'élément de capteur (110) comprend au moins deux contre-électrodes (116) agencées sur des côtés opposés de l'électrode de travail (114), dans lequel les contre-électrodes (116) sont sensiblement également espacées de l'électrode de travail (114).
